# EUROPEAN PATENT SPECIFICATION

(11) **EP 0 847 530 B1**
(45) Date of publication and mention of the grant of the patent: **16.06.2004**
(21) Application number: 96928295.3
(22) Date of filing: 30.08.1996
(51) Int. Cl.: G01N 33/68

(54) **QUANTITATION OF p97 TO DIAGNOSE AND MONITOR ALZHEIMER'S DISEASE**
QUANTIFIZIERUNG VON p97 ZUR DIAGNOSE UND ÜBERWACHUNG DER ALZHEIMHER-KRANKHEIT
QUANTIFICATION DE p97 POUR DIAGNOSTIQUER ET SURVEILLER LA MALADIE D'ALZHEIMER

(30) Priority: 31.08.1995 US 520933
(43) Date of publication of application: 17.06.1998
(73) Proprietor: The University of British Columbia, Vancouver, British Columbia, V6T 1Z3 (CA)
(72) Inventor: JEFFERIES, Wilfred, A., South Surrey, British Columbia V4A 2V5 (CA); KENNARD, Malcolm, Vancouver, British Columbia V6R 2J6 (CA)
(74) Representative: Sunderland, James Harry
(86) International application number: PCT/CA1996/000587
(87) International publication number: WO 1997/008560

(56) References cited:
- WO-A-94/01463
- NAT. MED., vol. 2, no. 11, 1996, N.Y, pages 1230-1235, XP000613536 M.L. KENNARD ET AL.: "Serum levels of the iron binding protein p97 are elevated in Alzheimer's disease."
- BRAIN RESEARCH, vol. 712, no. 1, 1996, pages 122-126, XP000613250 W. A. JEFFERIES ET AL.: "Reactive microglia specifically associated with amyloid plaques in Alzheimer's disease brain tissue espress melanotransferrin." cited in the application

## Description

The present invention relates to methods for quantitating p97 which are used to diagnose and monitor Alzheimer's Disease.

### BACKGROUND OF THE INVENTION

Alzheimer disease (AD) is a neurodegenerative disease that affects cognition, behaviour and function. In a recent study it was shown that AD affects almost 7% of individuals by the age of 75, increasing to over 1 in 4 by the age of 85 [Canadian Study of Health and Aging Working Group, *J. Can. Med. Assoc.* 150, 899-913 (1994)]. Other studies have claimed even higher incidences of AD affecting the aged [Evans, D.A. *J. Am. Med. Assoc.* 262, 2551-2559 (1989)]. At present there are only very limited therapeutic options for AD and the only definitive method for diagnosis is by brain autopsy.

AD is characterized by various pathological markers in the brain including senile plaques mainly composed of β-amyloid protein (Aβ), neurofibrillary tangles with hyperphosporylated microtubulin-associated protein Tau [Goedert, M., Spillantini,et al., *Neuron* 8, 156-160 (1992), and Selkoe, D.J. *Neuron* 6, 487-498 (1991)], neuronal cell death and loss of synaptic connections [Terry, R. D., et al *Ann. Neurology* 30, 572-580 (1991)]. It has been proposed that abnormal deposition of the protein Aβ results in neuronal cell death in regions of the brain involved in cognition and memory [Blass, J.P. *Neurology* 43, S25-38 (1993); and Price, D.L.*Ann. Rev. Neurosci.* 9, 489-512 (1986)].

Clinically the diagnosis of AD is made through neurological and neuropathological assessments that unfortunately are unable to detect the disease in its early stage. The application of uniform clinical diagnostic criteria, such as NINCDS-ADRDA, has improved the accuracy of clinical pathologic diagnosis to over 80% [McKhann, G., *et al. Neurology* 34, 939-944 (1984)].

Attempts to correlate levels of cerebral spinal fluid (CSF) proteins with AD has met with limited success. For example, a test developed to detect Aβ in the CSF showed that total Aβ levels in AD patients did not differ significantly from controls [Shoji, M. *et al. Science* 258, 126-129 (1992)], although early-onset AD patients had slightly higher Aβ levels than elderly controls [Nakamura, T., *et al. Ann. Neurol*. 36, 903-911 (1994)]. It has been noted, however, that Aβ extending to position 42, Aβ₁₋₄₂ predominated in both diffuse and senile amyloid plaques in AD brain tissue [Roher, A., *et al. J. Biol. Chem*. 268, 3072-3083 (1993)] and that A β₁₋₄₂ was found to be significantly lower in the CSF of AD patients relative to controls [Motter, R., *et al. Ann. Neurol.* 38, 643-648 (1995)]. In studies on the secreted form of the amyloid precursor protein (APP), from which Aβ is derived, it was found that soluble APP was considerably reduced in the CSF of AD patients compared to controls [Van Nostrand, W.E. *et al. Proc. Natl. Acad. Sci. USA* 89, 2551-2555(1992)]. Other studies, however, have only noted slight decreases [Palmert, M.R., *et al. Neurol.* 40, 1028-1034 (1990)] or even increases in APP [Kitaguchi, N., *et al. Biochem. Biophys. Res. Comm.* 166, 1453-1459 (1990)]. Tests developed to monitor protein Tau in the CSF revealed that, although on average Tau levels were elevated in AD patients over controls, there was also considerable overlap with normal controls [Motter, R, *et al. Ann. Neurol.* 38, 643-648 (1995), Vigo-Pelfrey, C. *et al. Neurology* 45, 788-793 (1995)], and with controls suffering from other neurological diseases [Vandermeeren, M., *et al. J. Neorochem*. 61,1828-1834 (1993)].

Other CSF diagnostic markers have been considered and found unsuitable, such as alphal-antichymotrypsin associated with senile plaques [Abraham, C.R., Selkoe, D.J. & Potter, H. *Cell.* 52, 487-501 (1988)] and ubiquitin [Wang, G.P., *et al. Acta Neuropathol. (Berl.)* 82, 6-12 (1991)], due to contradictory results and overlap between AD and controls. Not only are these results disappointing, but routine sampling CSF for patient diagnosis is unlikely to be well received. However, a commercially available diagnostic test has been developed by Athena Neuroscience Inc. that combines measuring CSF Tau and Aβ₁₋₄₂ levels with apolipoprotein E (ApoE) E4 allele frequency located on chromosome 19 in humans. It has been established that ApoE is associated with senile plaques and that people who are homozygous for ApoE E4 have an increased probability of developing AD [Sanders, A.M., *et al.Neurol*. 43, 1467-1472 (1993)]. It is claimed that this combined analysis can help physicians determine the likelihood of a patient having AD [Motter, R., *et al. Ann. Neurol*. 38, 643-648 (1995)]. However, the test is time consuming and provides only a probable answer.

A recent report studying pupillary response has suggested that AD patients exhibit hypersensitivity to a dilute solution of the acetylcholine blocking drug, tropicamide [Scinto, L.F. *et al.. Science* 266, 1051-1054 (1994)]. This hypersensitivity was first noted in Down's syndrome subjects who invariably develop a neuropathology that is very similar to AD [Olson, M.I. & Shaw, C.M. *Brain* 92, 147-156 (1969)] by middle age. Although this study appeared promising the results have not been readily replicated and suffer from overlap, and difficulties in interpretation, such as the effect that eye disorders or colour may have on the test [Loupe, D.N., *et al. Opthalmology* 103, 495-503 (1996)]. Most recently a strategy was described [Parshad, R, *et al. Proc. Natl. Acad. Sci. USA* 93, 5246-5150 (1996)] where AD cells were identified by detecting defects in their ability to repair DNA damage. It was concluded that the test could prove useful in supporting or rendering unlikely the diagnosis of AD. However, the test is still far from clinical practice since it is laborious and requires multiple steps with cultured cells. Finally it has been noted that it is possible to detect variations in glucose metabolism within the brains of AD patients using positron emission tomography [Reiman, E.M., *N. Engl. J. Med.* 334, 752-758 (1996)]. The test is not practical for use on a routine basis.

Considerable interest has been directed at the genetic causes of AD and mutations in several genes have been shown to confer susceptibility to a small number of familial AD cases. Mutations in the APP gene [Murrell, J., et al., *Science* 254, 97-99 (1991) and Karlinsky,H.*Neurology* 42, 1445-1449 (1992)] on chromosome 21 have been correlated with autosomal dominant, early-onset (<65 years) AD and mutations in the presenilins, S182 linked to chromosome 14 [Schellenberg, G.D *et al. Science* 258, 668-671 (1992); Van Broeckhoven, C. *et al. Nature Genet* 2,335-339 (1992); and Sherrington, R. *et al. Nature* 375, 754-760 (1995)] and STM2 linked to chromosome 1 [Levy-Lahad, E., *et al. Science* 269, 973-977 (1995); and Rogaev, E.I., *et al. Nature* 376, 775-778 (1995)], have been related to a small number of cases of familial AD. In addition, the E4 allele of the ApoE gene found on chromosome 19 modifies the risk of developing the late-onset form of AD [Sanders, A.M., *et al.. Neurol.* 43, 1467-1472 (1993); and Rogaev, E.I., *et al. Nature* 376, 775-778 (1995)]. While discovery of these genes will have considerable value in determining the predisposition of a small number of individuals to develop AD, genetic assessment will not be useful in detecting or monitoring AD.

The p97 antigen, also known as melanotransferrin, has been associated with AD (PCT/CA93/00272 published as WO94/01463 on January 20, 1994). p97 belongs to the important group of iron binding proteins that include serum transferrin (Tf), lactoferrin and ovotransferrin from avian egg whites [Baker, E.N., Rumball, et al.,*Trends Biochem. Sci.* 12, 350-353 (1987)]. p97 is able to bind iron and is involved in cellular iron uptake [Kennard, M.L., et al., *EMBO J.* 14, 4178-4186 (1995)]. There are two forms of p97; one which is attached to the cell surface by a glycosyl-phosphatidylinositol anchor and one which is actively secreted [Food, M.R. *et al. J. Biol. Chem.* 269, 3034-3040 (1994)]. In a recent study p97 and the transferrin receptor (TR) were found to be highly localized to the capillary endothelium of human brain. Whereas transferrin (Tf) itself was found mainly localized to glial cells [Rothenberger, S. *et al.. Brain Res.* 712, 117-121 (1996)]. p97 was also shown to be specifically expressed on reactive microglia cells associated with amyloid plaques in post mortem brain tissue from AD patients [Jefferies, W.A. *et al.Brain Res.* 712, 122-126 (1996)]. All other microglia not associated with the senile AD plaques and those found in brain tissue from other neuropathologies (Parkinson disease, progressive supranuclear palsy, Huntington disease and amyotrophic lateral sclerosis) did not express detectable levels of p97.

### SUMMARY OF THE INVENTION

The present inventors have specifically shown that the soluble form of the iron binding protein, p97 is significantly elevated in the serum and cerebral spinal fluid (CSF) of Alzheimer's patients compared to healthy individuals. The amount of p97 in samples from Alzheimer's patients was consistently determined to be higher as compared with the amount of p97 in samples from healthy individuals. The present inventors have also significantly shown that p97 levels in serum increase with increasing duration of the disease. In addition, p97 levels appeared to begin to increase an estimated two years prior to observed symptoms of AD. The specific quantification of p97 can identify subjects afflicted with the disease and can be used to monitor the onset and longitudinal progression of the disease.

As a result of these findings the present inventors have designed a simple and reliable test for the detection of AD in body fluids which is a valuable tool in both the assessment and management of the disease. Early diagnosis of AD using the present invention give families more time to plan for the proper care of Alzheimer's patients and eliminates the possibility of conditions that mimic Alzheimer's symptoms, such as depression or stroke. The method of the present invention can also be used to monitor the effectiveness of new treatment strategies for AD. Although there are many drugs being developed for treatment of AD, there is no inexpensive and quick method to study the effectiveness of these drugs. Current clinical trials attempt to measure efficacy with complex and labour intensive neurobehavioural assessment.

Broadly stated the present invention relates to a method of diagnosing Alzheimer's Disease by quantitating p97 in a sample of body fluid from a patient comprising the steps of:
a) obtaining a sample of serum from a patient suspected of having Alzheimer's Disease, thereby obtaining a test sample;
b) determining the amount of p97 in the test sample; and
c) comparing the amount of p97 in the test sample with an amount of p97 in control samples,
wherein the presence of an amount of p97 in the test sample which is elevated as compared with the amount of p97 in the control samples is indicative of the potential for Alzheimer's Disease.

The specification also describes a method of monitoring the progression of Alzheimer's Disease by quantitating p97 in a sample of body fluid from a patient having Alzheimer's Disease comprising the steps of
a) obtaining a sample of body fluid from a patient having Alzheimer's Disease, thereby obtaining a test sample;
b) determining the amount of p97 in the test sample; and
c) comparing the amount of p97 in the test sample with an amount of p97 in a first test sample previously obtained from the patient,
wherein the presence of an amount of p97 in the test sample which is elevated as compared with the amount of p97 in the first test sample is indicative of the progression of Alzheimer's Disease in the patient.

Further the specification describes a method of monitoring a treatment of Alzheimer's Disease by quantitating p97 in a sample of body fluid from a patient having Alzheimer's Disease comprising the steps of
a) obtaining a sample of body fluid from a patient who has received a treatment for Alzheimer's Disease thereby obtaining a test sample;
b) determining the amount of p97 in the test sample; and
c) comparing the amount of p97 in the test sample with an amount of p97 in a pre-treatment sample obtained from the patient prior to the treatment,
wherein differences in the amount of p97 in the test sample as compared with the amount of p97 in the pre-treatment sample is indicative of the efficacy of the treatment.

The invention still further contemplates kits useful in performing the methods of the invention comprising an agent which detects the presence of p97 in a serum sample and all the reagents required to detect the presence of p97, and suitable supports useful in performing the methods of the invention.

These and other aspects of the present invention will become evident upon reference to the following detailed description and attached drawings. In addition, reference is made herein to various patent documents and publications, which are hereby incorporated by reference in their entirety.

### BRIEF DESCRIPTION OF THE DRAWINGS

Figure 1 is a calibration curve for p97 standards;
Figure 2 is a graph showing a comparison between serum p97 levels for Alzheimer's disease patients and control subjects based on age;
Figure 3 is a graph showing a comparison between serum p97 levels for Alzheimer's disease patients and control subjects based on duration of disease;
Figure 4 is a graph showing a comparison between serum transferrin levels for Alzheimer's disease patients and control subjects based on age;
Figure 5 is a graph showing a comparison of serum p97 concentrations from AD subjects and controls with subject age;
Figure 6 is a graph showing a comparison of serum p97 concentrations from AD patients with time since the patient was first observed with symptoms of AD;
Figure 7 is a graph showing a comparison of serum transferrin concentrations from AD subjects and controls with subject age; and
Figure 8 is a bar graph showing the ratio of serum p97 concentrations from AD and spousal control pairs.

### DETAILED DESCRIPTION OF THE INVENTION

The present invention provides methods for monitoring and diagnosing Alzheimer's Disease in a patient by quantitating p97 in a sample of body fluid from a patient. The method involves obtaining a test sample of body fluid from a patient. The term "patient" refers to a warm-blooded animal such as a mammal, preferably a human individual, which is afflicted with Alzheimer's disease or is suspected of being afflicted with Alzheimer's disease. The patient may or may not exhibit cognitive impairment, and the patient may be receiving a treatment for AD. Generally, the diagnostic method of the invention is used to determine whether an individual who does not exhibit any symptoms of AD has a predispostiion or potential to develop AD.

The test sample of the invention is serum, while it may be obtained from a variety of body fluids, including for example, lymph, bile, sputum or cerebrospinal fluid. Cell samples may also be used as test samples, such as blood cells, preferably monocytes. In particular, activated macrophages expressing p97 may be assayed. Preferably, the test sample is obtained from serum or CSF, most preferably serum. The test samples are obtained using known techniques.

In a particularly preferred embodiment blood serum samples are obtained from a patient. Samples may be stored and frozen (e.g. at -80°C) prior to use and may be used neat and/or diluted, for example in 50% v/v fetal calf serum (FCA) in Pandex buffer (DNEM containing 0.1% NaH3 and 1.0% w/v BSA).

p97 is quantitated in a test sample using an agent which allows the p97 to be quantitated in the test sample. Preferably the agent recognizes and binds p97 in a test sample. In an embodiment of the invention the agent is an antibody.

The term "antibody" used herein includes polyclonal and monoclonal antibodies; mixtures of more than one antibody reactive with p97 (e.g. a cocktail of different types of monoclonal antibodies reactive with p97); whole antibodies; biologically functional fragments thereof which are sufficient for binding of the antibody fragment to p97; and chimeric antibodies comprising portions from more than one species; bifunctional antibodies; and, tetrameric antibodies.

Conventional methods can be used to prepare the antibodies. For example, by using a peptide of p97 polyclonal antisera or monoclonal antibodies can be made using standard methods. A mammal, (e.g., a mouse, hamster, or rabbit) can be immunized with an immunogenic form of the peptide which elicits an antibody response in the mammal. Techniques for conferring immunogenicity on a peptide include conjugation to carriers or other techniques well known in the art. For example, the peptide can be administered in the presence of adjuvant. The progress of immunization can be monitored by detection of antibody titers in plasma or serum. Standard ELISA or other immunoassay procedures can be used with the immunogen as antigen to assess the levels of antibodies. Following immunization, antisera can be obtained and, if desired, polyclonal antibodies isolated from the sera.

To produce monoclonal antibodies, antibody producing cells (lymphocytes) can be harvested from an immunized animal and fused with myeloma cells by standard somatic cell fusion procedures thus immortalizing these cells and yielding hybridoma cells. Such techniques are well known in the art, (e.g., the hybridoma technique originally developed by Kohler and Milstein (Nature 256, 495-497 (1975)) as well as other techniques such as the human B-cell hybridoma technique (Kozbor et al., Immunol. Today 4, 72 (1983)), the EBV-hybridoma technique to produce human monoclonal antibodies (Cole et al. Monoclonal Antibodies in Cancer Therapy (1985) Allen R. Bliss, Inc., pages 77-96), and screening of combinatorial antibody libraries (Huse et al., Science 246, 1275 (1989)]. Hybridoma cells can be screened immunochemically for production of antibodies specifically reactive with p97 and the monoclonal antibodies can be isolated.

Alternatively, a SCID-hu mouse, for example the model developed by Genpharm, can be used to produce antibodies, or fragments thereof reactive with p97.

The antibodies may also be obtained from various sources including for example, laboratories or depositories such as the American Type Culture Collection. For example, anti-p97 mouse monoclonal Ab, Hyb C (33B6E4) can be obtained from Dr. Shuen-Kuei Liao, McMaster University, Hamilton, ON); anti-p97 monoclonal antibody 9B6 can be obtained from the Biotechnology Laboratory, UBC, BC, Canada, or the anti-p97 mouse monoclonal antibody, L235 can be obtained from the American Type Culture Collection (ATCC-HB 8446 L235 (H-19).

Various other agents which recognize and bind p97 in a test sample and allow its presence to be quantitated in the sample can be used in the method of the inventon. For example, the transferrin receptor binds to p97 and can be used to quantitate p97 in a test sample. In addition, p97 binds iron and other metals which can be used to quantitate p97 in a test sample using standard methods (see PCT/CA93/00272 published as WO94/01463 on January 20,1994 which describes iron binding assays).

Agents used in the methods of the invention can be detectably labelled with a detectable substance, or they can be subsequently detectably labelled. Examples of detectable substances include various enzymes, fluorescent materials, luminescent materials and radioactive materials. Examples of suitable enzymes include horseradish peroxidase, biotin, alkaline phosphatase, β-galactosidase, or acetylcholinesterase; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; and examples of suitable radioactive material include radioactive iodine I¹²⁵, I¹³¹ or tritium.

Agents used in the methods of the invention can be subsequently detectably labelled using for example a substance which recognizes and binds to the the agent. By way of example, if the agent is an antibody (e.g. mouse IgG antibody), a second antibody reactive with the agent (e.g. a rabbit anti-mouse gamma-globulin) which is labelled with a detectable substance as described herein, may be used to detect the agent thereby allowing quantitation of p97.

An agent which is an antibody may be used to detect and quantitate p97 in known immunoassays which rely on the binding interaction between an antigenic determinant of p97, and the antibody. Examples of such assays are radioimmunoassays, enzyme immunoassays (e.g.ELISA), immunofluorescence, immunoprecipitation, latex agglutination, hemagglutination, countercurrent immuno-electrophoresis (CIEP), radioimmunoprecipitations, Dot Blot assays, inhibition or competition assays and sandwich assays.

In a preferred embodiment an assay based on a rapid immunofluorescent technique, [e.g. "Particle concentration fluorescence immunoassay" (PCFIA) described in Jolley et al. 1984, J. Immunol. Meth., 67, 21-35] is used to quantitate or determine levels of p97 in a sample. This method employs capture antibodies (Ab) bound to sub-micron polystyrene beads. This "activated" solid phase acts as a specific absorbent for the protein of interest. A fluorescent labeled second Ab, also specific for the protein, is then incubated with the solid capture phase to form a complex whose fluorescent signal is proportional to the original protein concentration. The reactions may be carried out in specially designed 96 well plates (Catalog 22-400-1; Idexx Laboratories Inc., Wesbrook, ME). Each well contains a 0.22 µm cellulose acetate membrane that allows the wells to be drained under vacuum to concentrate the fluorescent complex in the base of each well. The plates may be washed and each well read for fluorescence at varying wavelengths using a Pandex Fluorescence Concentration Analyzer (FCA; Idexx).

Activated beads for use in the assay may be prepared using anti-p97 antibodies to coat carboxy polystyrene particles (0.77 µm, 0.25% v/v; Idexx). Suitable anti-p97 antibodies include the anti-p97 mouse monoclonal Ab, Hyb C (33B6E4; Dr. Shuen-Kuei Liao, McMaster University, Hamilton, ON), 9B6 (Dr. Wilf Jefferies, Biotechnology Laboratory, UBC, BC), or anti-p97 rabbit antisera (Dr. Wilf Jefferies, Biotechnology Laboratory, UBC, BC).

The fluorescently labelled second antibody may be prepared using the anti-p97 mouse monoclonal antibody, L235 (ATCC-HB8446 L235 (H-19)) or anti-p97 rabbit antisera (Dr. Wilf Jefferies, Biotechnology Laboratory, UBC, BC), fluoresceinated with fluorescein isothiocyanate (FITC).

A p97 standard may be prepared from p97, for example, p97 purified from the supernatant of phosphatidylinositol phospholipase C (PI-PLC) treated Chinese hamster ovary (CHO) cells, transfected with human p97), by immunoaffinity chromatography.

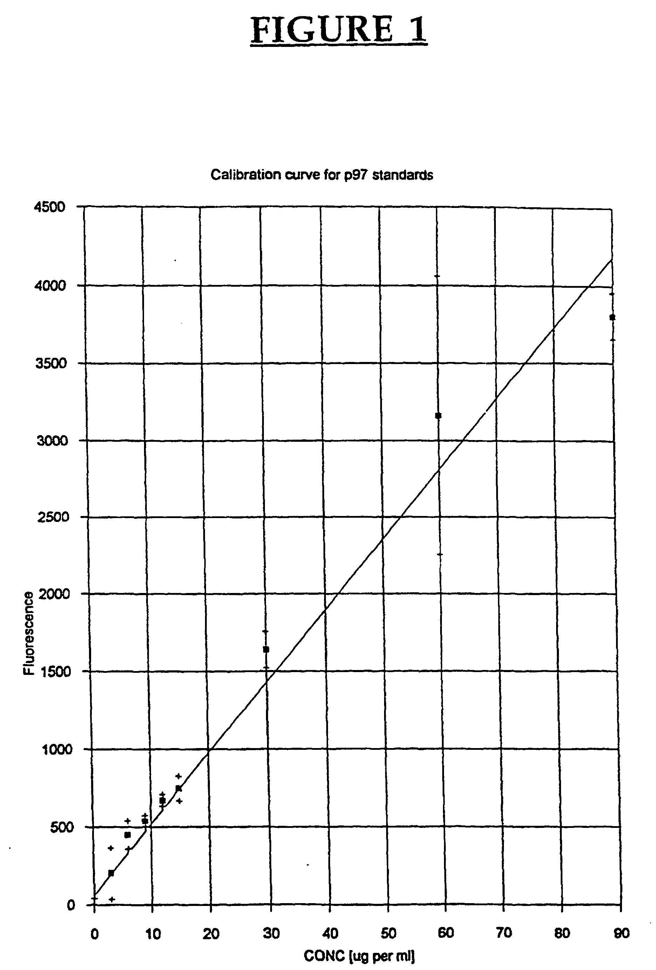

In a particularly preferred embodiment, a blood serum p97 assay may be carried out in special 96 well plates (22-401-1; Idexx) and the fluorescence read in the FCA (Idexx). 60µL of the blood-serum sample at the appropriate dilution in the 50% FCA solution may be added to a well on the 96 well plate. p97 standards may be used for calibration curve preparation. A sample calibration curve preparation is shown in Table 1 and a calibration curve is shown in Figure 1.

The present inventors have found that p97 levels begin to increase an estimated two years prior to observed symptoms of Alzheimer's Disease. Therefore, the method of the present invention may be used to diagnose Alzheimer's Disease, or potential for developing Alzheimer's Disease, in a patient which does not have clinically evident symptoms of Alzheimer's Disease, thereby providing an early prognosis for the disease. To diagnose Alzheimer's Disease, the concentration of p97 in the patient sample may be compared to a range of concentrations of p97 in control samples from healthy subjects that may be established by prospective and/or retrospective statistical studies. Healthy subjects may be selected based on NINCDS-ADRDA criteria and/or the results of MMS Tests. Preferably, the healthy subjects have no clinically evident cognitive impairment or other clinical or pathological problems. Diagnosis may also be made by a finding of increased levels of p97 compared to previous levels quantitated for the same patient.

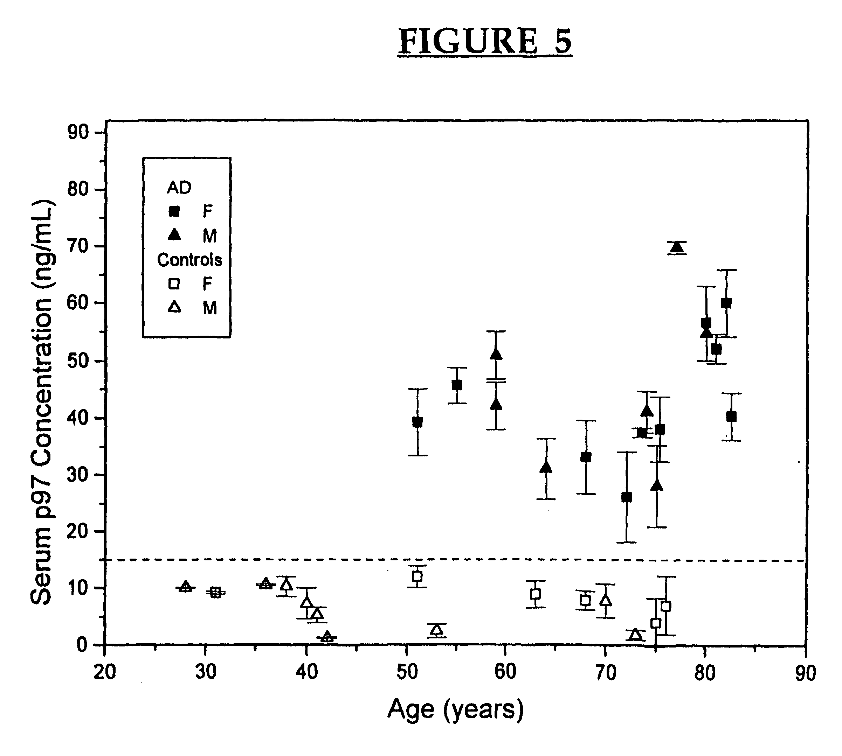

Elevated levels of p97 in a test sample compared to controls indicates that the patient has Alzheimer's Disease, or potential for developing Alzheimer's Disease. By way of example, levels of p97 in the serum of a representative number of control subjects are determined, the average p97 levels are determined, or p97 levels are plotted against the age of the subjects as shown in Figure 5 and a regression line or baseline (- - - in Figure 5) is established for the control subjects. A test sample containing p97 levels above the average or baseline is indicative of Alzheimer's Disease or the potential for Alzheimer's Disease. Generally, levels of p97 in a sample from an Alzheimer's Disease patient which are elevated one and one half fold or more, in particular two fold or more, preferably two to nine fold, over levels in the serum of control subjects, are indicative of the potential for Alzheimer's Disease.

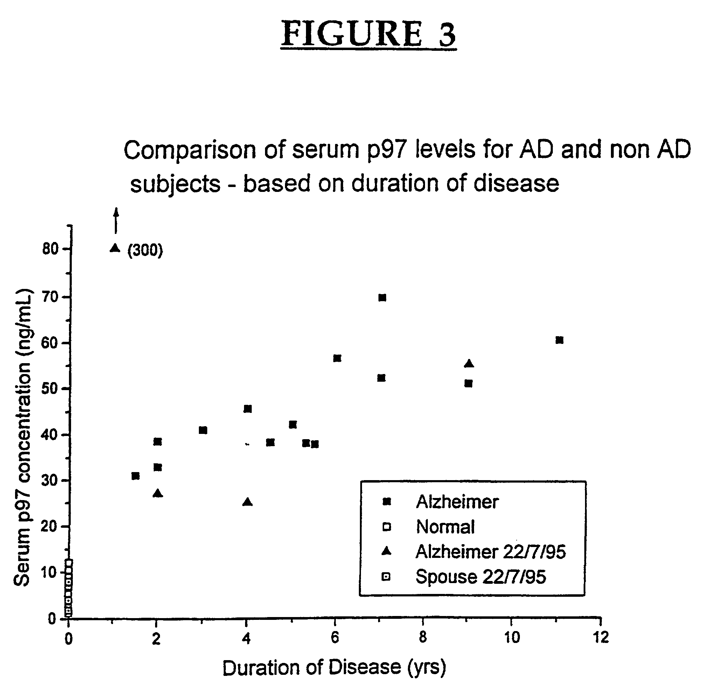

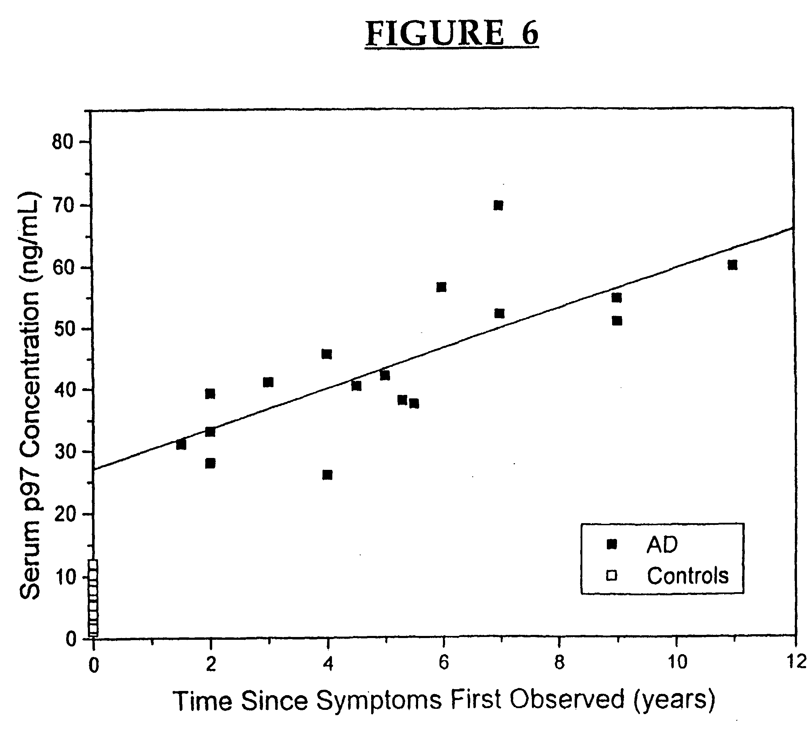

p97 levels in serum have been found to increase with progression of the disease (see Figures 3 and 6). Therefore, to monitor the progression of Alzheimer's Disease in a patient, the concentration of p97 in a sample from the patient may be compared to levels of p97 from previous samples from the same patient as described herein. Progression and assessment of the stage of the disease may also be determined by comparing the levels in a test sample with the levels obtained from control subjects as described herein, or levels obtained from other Alzheimer's Disease patients. This latter comparison is based on the linear relationship between p97 levels in samples from body fluids and the progression of the disease. By way of example, the stage of disease in a patient may be determined by quantitating p97 levels in a sample from the patient, and extrapolating from a standard curve (for example, the graph as shown in Figure 6).

The methods of the invention may also be used to monitor or assess in a patient the efficacy of a therapeutic treatment for Alzheimer's Disease. Samples may be taken prior to, during and/or after treatment and efficacy of the treatment determined by the affect of the treatment on the concentration of p97 in the samples. An effective treatment will be expected to be a treatment which results in lower levels of p97 in the samples compared to a control.

It is contemplated that the method may be used to monitor the efficacy of any type of treatment for Alzheimer's Disease, in particular the use of pharmaceutical compositions suspected of having efficacy in the treatment of Alzheimer's Disease. Examples of pharmaceutical compositions which may have some efficacy in the treatment of Alzheimer's Disease include substances which restore or replace cholinergic function, such as tacrine, choline, lecithin, huperzine A and B, galanthamine, methanesulfonyl fluoride, physostigmine and deprenyl.

The reagents suitable for applying the methods of the invention to quantitate p97 may be packaged into convenient kits providing the necessary materials packaged into suitable containers. For example, such kits may include antibodies which react with p97, and the necessary reagents for quantitating antibodies bound to p97 present in a sample by means of the methods described herein. The kits may also include suitable supports useful in performing the methods of the invention.

The following examples describe the present inventors' quantitation of p97 in samples of patients with Alzheimer's Disease and controls, and the implications of such quantification. The examples are offered by way of illustration, and not by way of limitation.

### EXAMPLES

### EXAMPLE 1

### STUDY RE p97 LEVELS IN THE SERUM OF ALZHEIMER PATIENTS

An assay for measuring levels of p97 in human blood serum was developed based on a rapid immunofluorescent technique, "Particle concentration fluorescence immunoassay" (PCFIA) introduced in 1984 (Jolley et al. 1984, J. Immunol. Meth., 67, 21-35). This method employs capture antibodies (Ab) bound to sub-micron polystyrene beads. This "activated" solid phase acts as a specific absorbent for the protein of interest. A fluorescent labeled second Ab, also specific for the protein, was then incubated with the solid capture phase to form a complex whose fluorescent signal was proportional to the original protein concentration. The reactions were carried out in specially designed 96 well plates (Catalog 22-400-1; Idexx Laboratories Inc., Wesbrook, ME). Each well contained a 0.22 µm cellulose acetate membrane that allowed the wells to be drained under vacuum to concentrate the flourescent complex in the base of each well. These plates were then washed and each well read for fluorescence at varying wavelengths using a Pandex Fluorescence Concentration Analyzer (FCA; Idexx).

Activated beads for use in the assay were prepared using the following antibodies to coat carboxy polystyrene particles (0.77 µm, 0.25% v/v; Idexx): the anti-p97 mouse monoclonal Ab, Hyb C (33B6E4; Dr. Shuen-Kuei Liao, McMaster University, Hamilton, ON), or 9B6 (Dr. Wilf Jefferies, Biotechnology Laboratory, UBC, BC), or anti-p97 rabbit antisera (Dr. Wilf Jefferies, Biotechnology Laboratory, UBC, BC). 1 mL of the particles (vortexed and sonicated for 1 min) were centrifuged and resuspended in 8 mL of 0.1 M MES [2(4-morpholino) ethanesulphonic acid] buffer pH 4.5. To this 5.0 mg of EDC [1-ethyl-3-(3-dimethylaminopropyl) carbodiimide] was added followed by 1mL of antibody (1 mg/mL). The mixture was vortexed periodically, incubated overnight at room temperature and centrifuged at 6000 rpm for 10 min (Sorval HB4) and the beads were resuspended in 20 mL of phosphate buffered saline (PBS) containing 0.2% sodium azide (NaN3) and 2% w/v bovine serum albumen (BSA). The beads were then centrifuged at 6000 rpm for 10 min and the coated beads were stored in 32 mL of PBS containing 0.2% NaN3 and 2% BSA at 4°C (∼concentration of antibody, 25µg/mL).

The fluorescently labelled second antibody was prepared using the anti-p97 mouse monoclonal antibody, L235 (ATCC-HB8446 L235 (H-19)) or anti-p97 rabbit antisera (Dr. Wilf Jefferies, Biotechnology Laboratory, UBC, BC), fluoresceinated with fluorescein isothiocyanate (FITC) as follows. FITC was added at 1mg/mL to phosphate buffer pH 9.5 (0.15 M Na2HPO4). In the absence of azide, 0.5 mL of the antibody at 4 mg/mL was added to the FITC solution (0.15 mL of FITC solution at 1 mg/mL) and incubated overnight at room temperature in the dark. The fluoresceinated Ab was ready for use and stored at 4°C.

A p97 standard was prepared from p97 purified from the supernatant of phosphatidylinositol phospholipase C (PI-PLC) treated Chinese hamster ovary (CHO) cells, transfected with human p97, by immunoaffinity chromatography. Approximately 10⁹ transfected CHO cells were treated with 1 mL of PI-PLC (300 mU/mL) in PBS for 1 hr at 37°C. The supernatant was recovered from the cells by centrifugation and filtered through a 0.2 µm membrane. The supernatant was then applied to a column (1 x 10) of Ab (HybC) immobilized on Affi-Gel 10 (Bio-Rad, Mississauga, ONT). The column had been previously washed and regenerated in PBS at pH 7.2. Bound p97 was eluted with 0.1 M citric acid, pH 3.0, followed by neutralization with 1 M Tris-HCL, pH 9.0. The purified p97 was concentrated using a 30,000 MW ultrafiltration membrane. Dialysed against PBS and sterile filtered. The concentration of the standard p97 was determined using the p97 extinction coefficient at 280 nm 1%=12.0 cm-1 (Baker et al. 1992).

Blood serum samples were prepared as follows. For each patient the following samples were taken: (a) serum sample stored at -20°C and (b)fresh blood stored at 4°C. Before testing the samples, the fresh blood was centrifuged and the serum recovered. Both types of sample were tested neat and/or diluted in 50% v/v fetal calf serum (FCA) in Pandex buffer (DNEM containing 0.1% NaH3 and 1.0% w/v BSA).

The blood serum p97 assay was carried out in the special 96 well plates (22-401-1; Idexx) and the fluorescence read in the FCA (Idexx) as follows. 60µL of the blood serum sample at the appropriate dilution in the 50% FCA solution was added to a well on the 96 well plate. Each sample was tested in duplicate or triplicate. To each plate p97 standards were also added in duplicate (300, 150, 120, 90, 60, 30, 15, 9, 6 ng/mL). These standards were diluted in the 50% FCA solution and used for calibration curve preparation. A sample calibration curve preparation is shown in Table 1 and a calibration curve is shown in Figure 1. 20µL of the anti-p97 coated beads (-25 µg Ab/mL) was added to each sample and incubated at room temperature for 40 min. The contents of the wells were gently mixed by tapping the sides of the 96 well plate. Following the incubation, 20 µL of the fluoresceinated second anti-p97 Ab was added (diluted 1/75 in Pandex buffer (∼25-40 µg/mL)) to the sample and beads and incubated for 5-10 min at room temperature. The plates were then placed in the FCA, drained and washed 3-5 times in PBS containing 0.1% NaH3 and 1% w/v BSA. The drained plates were then read with the 485/535 nm filter pair at 25x gain.

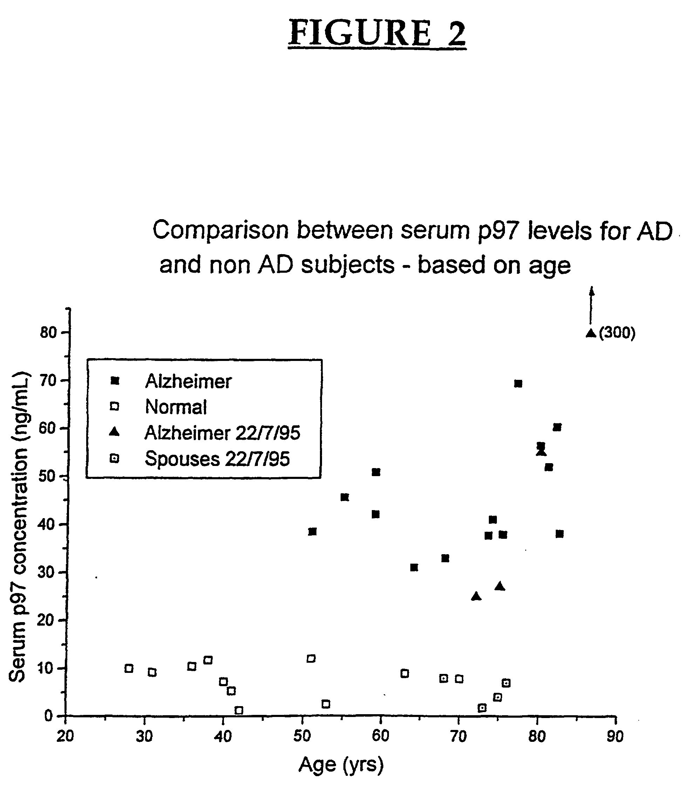

Blood serum samples were obtained from Alzheimer (AD) patients, from spouse controls and from unrelated controls. Table 2 shows sample results of p97 blood serum concentrations of p97 in AD patients. Duration of the disease indicates the number of years since diagnosis of the condition. However, it is quite possible that an individual patient had been suffering from the disease for some time prior to diagnosis. Table 3 shows the p97 blood serum concentrations in AD patient and control samples. Levels of p97 in the serum of unrelated controls ranged between 2.4 to 12 ng/ml and levels were found not to increase with age of the subject (Figure 2). As shown in Figure 2, levels of p97 in the serum of AD patients was significantly elevated compared to the controls and levels appeared to increase with age of the patient. AD patients had levels of p97 in the serum of at least 20 ng/ml. The maximum level found was 300 ng/ml. Importantly, serum p97 levels were found to be correlated with duration of disease in AD patients as shown in Figure 3. Increasingly higher levels of p97 were found in the serum of patients with longer duration of disease.

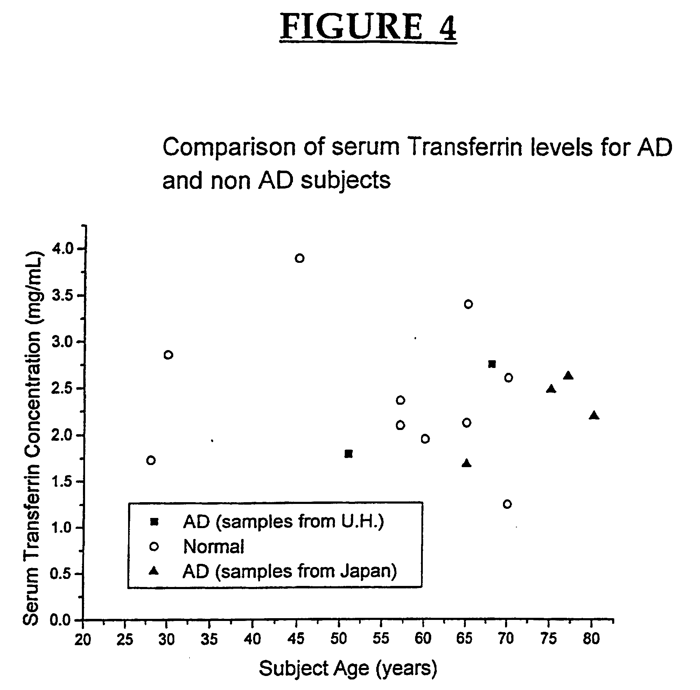

Serum transferrin levels were also measured in samples from AD patients and controls. No apparent difference was found in serum transferrin levels between AD patients and controls and no correlation was found between age of the subject and serum levels of transferrin (Figure 4).

Transferrin and p97 levels were also measured in CSF and serum samples previously obtained from a group of Japanese AD patients and control subjects. These samples had been frozen for two years and subjected to thawing and refreezing, thus the actual levels of protein may not reflect the absolute levels originally present in the samples. However, the results, shown in Table 4 confirmed the above findings that p97 levels were elevated in the serum of AD patients compared to levels in control subjects. The results also indicated that p97 levels in CSF were elevated in AD patients compared to controls. Transferrin levels in the serum and CSF of the AD patients were not elevated over control levels.

### EXAMPLE 2

A more complete description and discussion of the studies illustrated in Example 1 are provided in this Example 2. The following materials and methods were used in the studies described in Example 2:
**Canadian Subjects.** The AD (N=27) subjects were selected from those attending the Clinical Trials Programme of the UBC Clinic for Alzheimer disease and related disorders, Vancouver Hospital. All AD subjects were diagnosed as "clinically probable" according to the NINCDS-ADRDA criteria. The estimated duration of cognitive symptomatology was determined for all AD subjects. Subjects were not on experimental medication at the time of this study. The controls, either randomly chosen from healthy volunteers (N=15) or spousal caregivers (N=10), demonstrated no clinically evident significant cognitive impairment.

Two studies were carried out: a) Serum samples were stored at 4°C immediately after venipuncture and were assayed for p97 and Tf within 24 hr. 17 AD subjects (10 female, 7 male) with ages ranging from 51 to 82.5 years (66.4±17.52 yr) were compared with 15 control subjects (6 female, 9 male) with ages ranging from 28 to 76 years (52.33±16.5 yr). b) Serum samples drawn from spousal pairs (N=10 pairs) were stored frozen at -20°C immediately after venipuncture. Samples were drawn from spousal pairs at the same time, frozen and then analyzed at the same time. The AD patients (7 female, 3 male) with ages ranging from 54 to 86 years (70.6±10.47 yr) were compared with their non-AD spouses (3 female, 7 male) with ages ranging from 53 to 84 years (69.9±10.21 yr).
**Japanese subjects.** Eight AD subjects (6 female, 2 male) were tested with ages ranging from 61 to 80 years (71.5±6.52 yr) and compared with seven control subjects (4 female, 3 male) with ages ranging from 57 to 72 years (66.57±6.4 yr). The serum and CSF samples from AD subjects and controls were obtained from Department of Neurology, School of Medicine, Chiba University. All AD subjects were diagnosed as "clinically probable" according to the NINCDS-ADRDA criteria. The controls came from elderly subjects suffering from the following neuropatholgies: 1 Parkinson disease, 2 spino-cerebellar degeneration, 1 amyotrophic lateral sclerosis, 2 cervical spondylosis and 1 peripheral neuropathy. The serum and CSF samples were frozen at -20°C immediately after drawing and collectively and identically thawed prior to analysis.
**p97 assay.** Samples were tested neat and diluted in 50% v/v fetal calf serum in DMEM buffer containing 0.1% sodium azide and 1.0% w/v bovine serum albumin. The anti-p97 mouse antibody, HybC was used to coat the carboxypolystyrene (0.77 µm) capture particles and the anti-p97 mouse monoclonal antibody, L235 (ATCC-HB 8446 L235 (H-19)) was fluoresceinated (Kennard, M.L., EMBOJ. 14, 4178-4186 (1995). Standards of p97 were freshly prepared in the DMEM buffer containing fetal calf serum in the range 300 to 1 ng/mL. The assay consisted of mixing 60 µL of samples and standards with 20 µL of capture particles (∼25 µg antibody/mL) in the specialized 96-well plates and incubating for 40 min at room temperature. Next, 20 µL of the flouresceinated secondary antibody (-25 to 40 µg/mL) was added and the mixture incubated for a further 5 to 10 min at room temperature. The plates were then placed in a "Pandex Fluorescence Concentration Analyzer" (Jolley, M.J. Immunol. Methods 67, 21-35 (1984)), drained and washed up to four times with PBS containing 0.1% sodium azide and 1.0% bovine serum albumin. The drained plates were then read with the 485/535 filter pair at 25x gain. p97 concentrations were determined from a calibration curve prepared from the p97 standards which correlated fluorescence with p97 concentration. All samples were assayed in triplicate at various dilutions and the concentration averaged from all results.
**Transferrin assay.** This assay was based on the previously described "Particle Concentration Fluorescence Immunoassay". Anti-human transferrin goat antisera was coated on the capture particles and anti-human transferrin sheep antisera was fluoresceinated. Transferrin standards were freshly prepared in the range of 3 to 0.5 µg/mL. All samples were assayed in triplicate at various dilutions and the concentration averaged from all results.

A quantitative assay was developed that could monitor the concentration of p97 in human bodily fluids. The assay was based on a rapid immunofluorescent technique, "Particle Concentration Fluorescence Immunoassay", which employs capture antibodies bound to sub-micron polystyrene beads and fluorescent labelled secondary antibodies (Kennard, M. L. et al., Biotech. Bioeng. 42, 480-486, 1993). The modified sandwich assay was carried out in specially designed 96 well plates that contain 0.22 µm cellulose acetate membranes. The wells can be drained under vacuum allowing the fluorescent complex to be concentrated at the base of each well. The plates can be washed and each well read for fluorescence where the fluorescence is proportional to the p97 concentration. p97 was found to be partially unstable in human serum and lost antigenicity with storage. Table 5 shows the effect of storage temperature and time on the detectable concentration of p97 spiked in serum initially at 100 ng/mL. p97 was rendered undetectable in samples that were heat shocked at 60°C for 30 min (surprisingly serum Tf appeared unaffected by this treatment) and samples that were stored at room temperature lost up to 20% antigenicity over 48 hr. However, samples were relatively stable over a 48 hr period when stored at 4°C and -20°C, although the freezing and thawing further reduced the detection of p97. For these reasons, in a study where the serum p97 concentration from AD patients was compared with cognitively normal controls, the serum samples were stored at 4°C immediately after drawing and assayed within 24 hr. Figure 5 shows that all the AD patients had elevated levels of p97 in their serum compared to controls and there was no overlap. The mean p97 concentration of the AD group (N=17, 43.8±11.6 ng/mL) was significantly different from the mean of the control group (N=15, 7.04±3.28 ng/mL) based on paired t test (t_{0.05}=12.96, p=6.6x10⁻¹⁰). To date there has been only one other study of p97 in human blood (Brown, J.P. et al., Proc. Natl. Acad. Sci. USA 78, 539-543, 1981) when p97 was detected in the range 1.3 to 2.7 ng/mL. Although the mean age of the control group (52.3±16.5 yr) was younger than the mean age of the AD group (66.4±17.52 yr), linear regression showed that there was no significant correlation between p97 serum concentration and subject age (AD patients: N=17, slope of regression line=0.359, R=0.30, p=0.249; Controls: N=15, slope of regression line=-0.07, R=-0.35, p=0.197). Furthermore, when the data for the AD patients was plotted against time since the patient was first observed with symptoms of AD (Figure 6), linear regression showed that there was a significant correlation between increased p97 serum concentration and the progression of the disease (N=17, slope of regression line=3.3, R=0.82, p=0.0003). Finally, the extrapolation of the linear regression to the maximum p97 concentration of the controls, suggested that the p97 concentration may begin to increase an estimated two years prior to observed symptoms of AD.

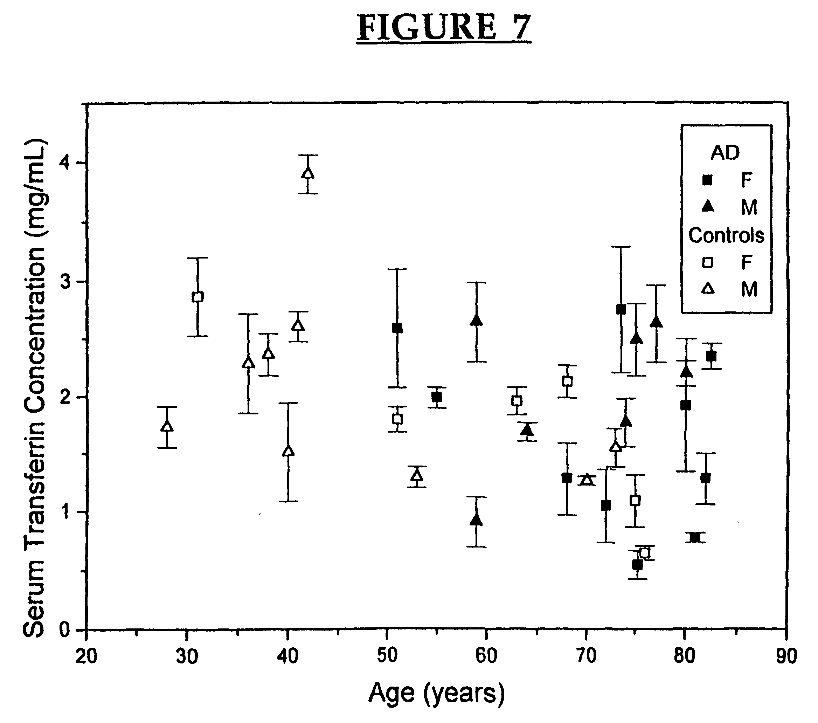

In order to eliminate the possibility that p97 non-specifically increases in AD, another blood iron binding protein, transferrin (Tf) was analyzed in the serum of AD patients and controls. Figure 7 shows that there was little difference between the Tf concentrations of both populations. The mean Tf concentration of the AD group (N=17, 1.81±0.71 mg/mL) was not significantly different from the mean of the control group (N=15, 1.93±0.78 mg/mL) based on paired t test (t_{0.05}=0.41, p=0.69).

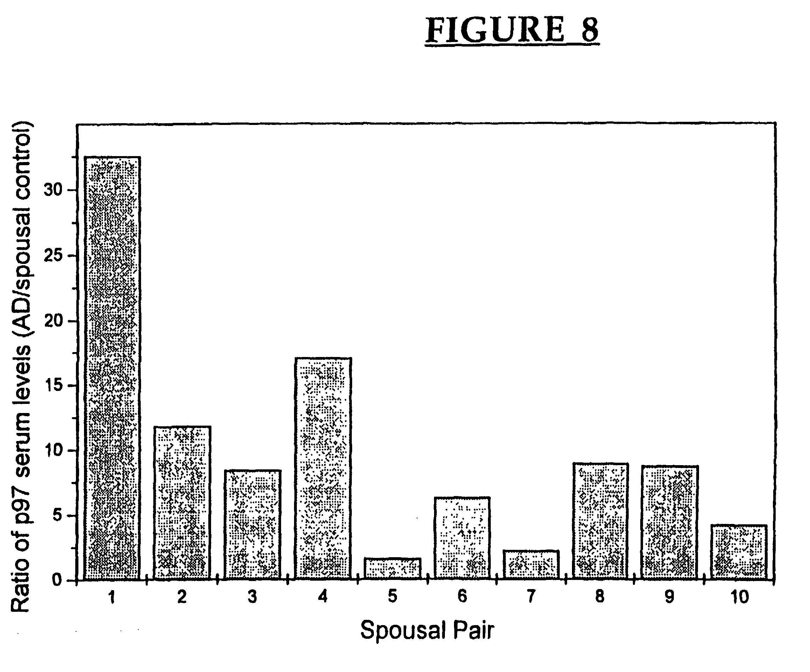

In another study the serum levels of p97 from AD patients was compared with their cognitively normal spouses to determine whether there might be an effect of diet, lifestyle or some other common factor on p97 concentration in serum. In this case the serum samples were immediately frozen at -20°C after drawing. Figure 8 compares the ratios of serum p97 levels from 10 pairs of AD patients (70.6±10.47 yr) and their spousal controls (69.9±10.21 yr). In all cases the p97 serum levels of AD patients were elevated compared to their spousal controls with the ratio ranging from 1.6 to 32.5 (mean 10.17±9.08). This finding provides evidence that environmental factors are probably not the cause of elevated p97 serum levels of AD patients.
**p97 and transferrin levels in serum and CSF**
In a third study, frozen samples of both serum and CSF from AD patients and controls of Japanese origin were analyzed for Tf and p97. The controls used in this study were subjects suffering from various other neuropathologies and were tested in order to determine whether serum levels of p97 were elevated in other neurodegenerative diseases. These serum samples, however, had been frozen and collectively and identically thawed prior to analysis, which unfortunately decreased p97 detection. Nevertheless, as can be seen in Table 6, several observations were worth noting. The mean concentration of p97 was elevated in the CSF of AD patients (N=5, 22.4±9.21 ng/mL; mean age 72.4±5.99 yr) compared to the controls (N=5, 8.48±4.02 ng/mL; mean age 67.2±6.82 yr). These concentrations were significantly different based on paired t test (t_{0.05}=2.90, p=0.044). This was also true for p97 in the serum where the mean p97 concentration of the AD group (N=4, 11.3±2.76 ng/mL; mean age 74.3±5.63 yr) was significantly different from the mean of the control group (N=6, 2.01±1.75 ng/mL; mean age 65.6±6.52 yr) based on paired t test (t_{0.05}=4.52, p=0.02). This was consistent with the data in Figure 5, although the overall concentrations were considerably reduced. Reinforcing the other observations, the p97 concentrations did not appear to correlate with age or sex of the subject. These data also suggest that monitoring the CSF, which has 2 to 4 fold higher than levels of serum p97, may provide valuable information regarding the onset and progression of AD. The mean concentration of Tf, which is considerably more stable in serum than p97, was virtually the same for both AD and control subjects in CSF and serum. For CSF the mean Tf concentration of the AD group (N=8, 20.35±4.78 µg/mL; mean age 71.5±6.52 yr) was not significantly different from the mean of the control group (N=7, 16.0±4.5 µg/mL; mean age 67.7±6.32 yr) based on paired t test (t_{0.05}=2.05, p=0.18). For serum, the mean Tf concentration of the AD group (N=4, 2.24±3.6 mg/mL; mean age 74.3±5.63 yr) was also not significantly different from the mean of the control group (N=5, 2.26±7.0 mg/mL; mean age 63.4±4.5 yr) based on paired t test (t_{0.05}=0.38, p=0.72). It is also interesting to note that the serum Tf levels were considerably higher than in the CSF (∼100 fold), which is in contrast to p97 whose levels are lower in serum than in the CSF. These data may imply that p97 has a unique function within the brain since it appears that Tf is actively excluded from the brain whereas p97 is not.

In this study a biochemical marker molecule has been identified that is consistently elevated in the serum of AD patients versus related and non-related controls. The present inventors have found no overlap between AD patients and controls.

From the foregoing, it will be appreciated that, although specific embodiments of the invention have been described herein for purposes of illustration, various modifications may be made without deviating from the spirit and scope of the invention. Accordingly, the invention is not limited except as by the appended.

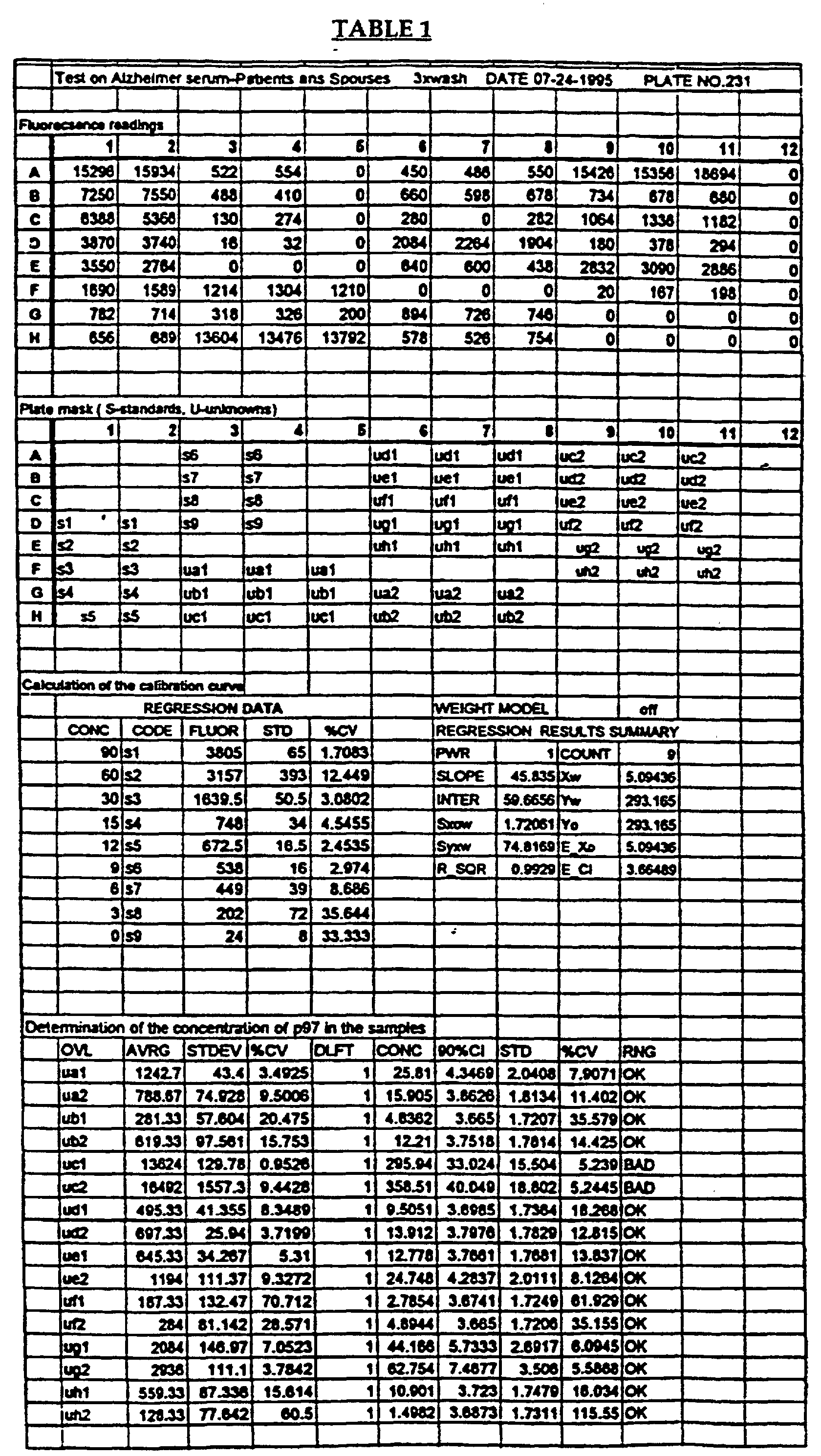

**TABLE 2**

| Blood serum tests for p97 concentration from Ad patients and normal controls | | | | | |
|---|---|---|---|---|---|
| AD Subjects: | | | | | |
| Subject | M/F | Date | Age (yr) | Duration (yr) | p97 conc (ng/mL) |
| 1 | F | 4/11/94 | 51 | 2 | 42.7 |
| 1 | F | 7/11/94 | 51 | 2 | 40.66 |
| 1 | F | 10/11/94 | 51 | 2 | 38.5 |
| 2 | F | 10/11/94 | 68 | 2 | 41.25 |
| 3 | F | 30/11/94 | 75 | 5 | 38.7 |
| 2 | F | 30/11/94 | 68 | 2 | 34.7 |
| 4 | F | 30/11/94 | 82 | 11 | 60.4 |
| 5 | F | 30/11/94 | 82.5 | 4.5 | 38.2 |
| 6 | M | 30/11/94 | 64 | 1.5 | 31 |
| 7 | F | 30/11/94 | 73.5 | 5.5 | 37.7 |
| 8 | F | 30/11/94 | 81 | 7.0 | 52 |
| | | | | | |
| 5 | F | 15/4/94 | 82 | 4 | 87.1 |
| 6 | M | 20/4/94 | 63.5 | 1 | 47.2 |
| 9 | M | 27/4/94 | 74 | 3 | 41 |
| 10 | M | 18/3/94 | 59 | 9 | 50.8 |
| 11 | M | 8/6/94 | 77 | 7 | 69.6 |
| 8 | F | 15/7/94 | 80.5 | 6.5 | 56.4 |
| 12 | F | 29/7/94 | 55 | 4 | 45.6 |
| 13 | M | 4/5/94 | 59 | 5 | 40.6 |
| 13 | M | 21/9/94 | 59.5 | 5.5 | 42 |

**TABLE 3**

| Samples tested 21 and 24 /7/95 - patients and spouse controls | | | | | | |
|---|---|---|---|---|---|---|
| Subject | M/F | Date | Age (yr) | Duration (yr) | P97 Concentration (ng/mL) | |
| | | | | | Blood | Serum |
| 1 | M | 21/7 | 75 | 2 | 28 | 24 |
| 1-spouse | F | 21/7 | 75 | - | 3 | 2 |
| 2 | M | 21/7 | 86 | 1 | 321 | 145 |
| 2-spouse | F | 21/7 | 76 | - | 3 | 4 |
| 3 | F | 21/7 | 72 | 4 | 25 | 22 |
| 3-spouse | M | 21/7 | 73 | - | 1 | 1 |
| 4 | M | 21/7 | 80 | 9 | 43 | 50 |
| 4-spouse | F | 21/7 | 68 | - | 7 | 3 |
| 1 | M | 24/7 | 75 | 2 | 26 | 20 |
| 1-spouse | F | 24/7 | 75 | - | 3 | 9 |
| 2 | M | 24/7 | 86 | 1 | 365 | 374 |
| 2-spouse | F | 24/7 | 76 | - | 9.5 | 12 |
| 3 | F | 24/7 | 72 | 4 | 28.3 | 27 |
| 3-spouse | M | 24/7 | 73 | - | 2 | 3 |
| 4 | M | 24/7 | 80 | 9 | 52.3 | 76 |
| 4-spouse | F | 24/7 | 68 | - | 9.4 | 12 |

| Unrelated Control Subjects | | | | | | |
|---|---|---|---|---|---|---|
| Subject | Age (yr) | p97 Concentration (ng/mL) | | | | |
| 1 | 31 | 9.2 | | | | |
| 2 | 28 | 10 | | | | |
| 3 | 36 | 10.5 | | | | |
| 4 | 38 | 11.8 | | | | |
| 5 | 40 | 7.3 | | | | |
| 6 | 41 | 5.3 | | | | |
| 7 | 42 | 1.2 | | | | |
| 8 | 51 | 12 | | | | |
| 9 | 53 | 2.4 | | | | |
| 10 | 63 | 8.9 | | | | |
| 11 | 70 | 7.8 | | | | |

**TABLE 4**

| Cerebral Spinal Fluid (CSF) and Blood Serum Samples from Japanese subjects Tested for Transferrin (Tf) and p97 | | |
|---|---|---|
| CSF | | |
| Alzheimer Subject | Tf conc. (µg/mL) | p97 Conc. (ng/mL) |
| 1 | 13.63 | - |
| 2 | 23.34 | - |
| 3 | 28.21 | 14.2 |
| 27 | 15.8 | - |
| 30 | 25.6 | 40.4 |
| 38 | 21.3 | 20.5 |
| 67 | 16.9 | 18.6 |
| 79 | 18 | 18.5 |

| Control | | |
|---|---|---|
| 4 | 18.0 | 11 |
| 5 | 12.4 | 11 |
| 6 | 9.9 | - |
| 7 | 12.2 | 10 |
| 8 | 23.4 | 0.5 |
| 9 | 13.9 | 9.9 |

| SERUM | | |
|---|---|---|
| Alzheimer Subject | Tf Conc, (mg/mL) | p97 Conc, (ng/mL) |
| 1 | 2.19 | 8.8 |
| 3 | 2.62 | 8.2 |
| 27 | 1.68 | - |
| 30 | 2.48 | 14.0 |

| Control | | |
|---|---|---|
| 4 | 3.4 | 0.1 |
| 5 | 2.06 | 0.8 |
| 6 | 1.25 | 2.0 |
| 7 | 2.12 | 2.1 |
| 10 | 1.95 | 5.6 |

**TABLE 5**

| **Stability* test of p97 in human serum (initially 100 ng p97/mL serum)** | | | |
|---|---|---|---|
| Temperature (°C) | Storage time (hr) | | |
| | 0.5 | 24 | 48 |
| 60 | 0 | nd | nd |
| Room temp. | 95.9 | 88.2 | 81.2 |
| 4 | 99.5 | 99.0 | 97.5 |
| - 20 | 94.0 | 92.4 | 91.2 |

| | | | |
|---|---|---|---|
| * Stability shown as a percent change in detectable p97 over time nd - not determined | | | |

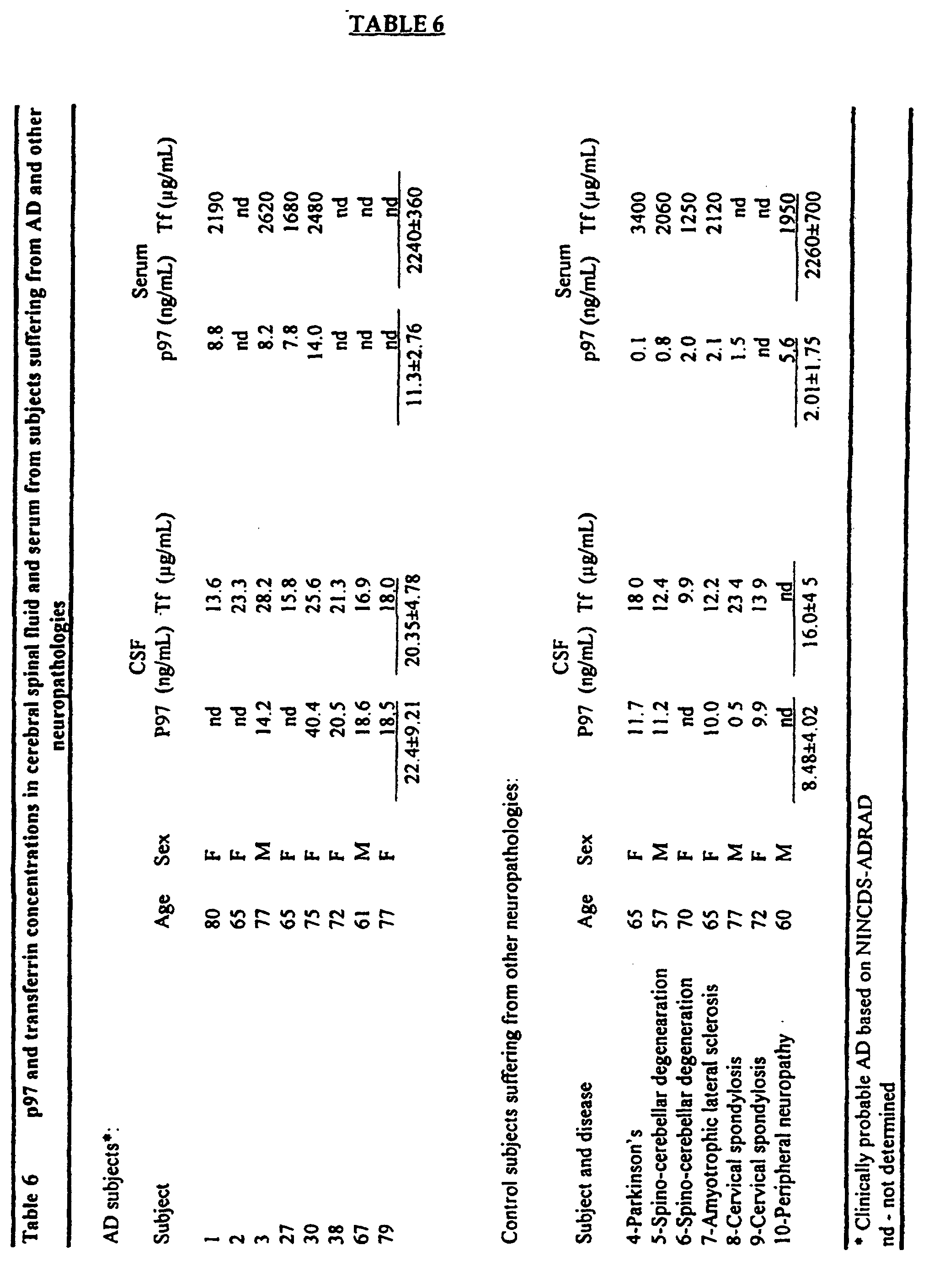

## Claims

1. A method for diagnosing Alzheimer's Disease by quantifying p97 in a test sample of serum obtained from a patient suspected of having Alzheimer's Disease comprising the steps of:
a) determining the amount of p97 in the test sample; and
b) comparing the amount of p97 in the test sample with an amount of p97 in control samples;
wherein the presence of an amount of p97 in the test sample which is elevated one and one half fold or more as compared with the amount of p97 in the control samples is indicative of the potential for Alzheimer's Disease.

2. A method as claimed in claims 1 wherein the amount of p97 in the test sample is elevated two to nine fold as compared to the amount of p97 in the control samples is indicative of the potential for Alzheimer's Disease.

3. A method as claimed in claim 1 wherein in step (b) the amount of p97 in the test sample is compared with an average or baseline amount of p97 determined in control samples.

4. A method as claimed in claim 1 wherein the control sample in (b) is a normal sample from the same patient or another individual.

5. A method as claimed in any one of claims 1 to 4 wherein the amount of p97 in step (a) is determined using antibodies to p97.

## Patentansprüche

1. Verfahren zur Diagnose der Alzheimer-Krankheit, indem in einer Untersuchungsprobe mit Serum von einem Patienten, bei dem ein Verdacht auf einer Alzheimer Erkrankung besteht, die Menge an p97 bestimmt wird, umfassend die Schritte:
a) Bestimmen der Menge an p97 in der Untersuchungsprobe; und
b) Vergleichen der Menge an p97 in der Untersuchungsprobe mit der Menge an p97 in Kontrollproben;
wobei die Anwesenheit einer Menge an p97 in der Untersuchungsprobe, die um das Eineinhalbfache oder mehr zur Menge an p97 in den Kontrollproben erhöht ist, ein Anzeichen für das Potential einer Alzheimer-Erkrankung ist.

2. Verfahren nach Anspruch 1, wobei eine zur Menge an p97 in den Kontrollproben um das zwei- bis neunfache erhöhte Menge an p97 in der Untersuchungsprobe ein Anzeichen für das Potential einer Alzheimer-Erkrankung ist.

3. Verfahren nach Anspruch 1, wobei im Schritt b) die Menge an p97 in der Untersuchungsprobe mit einer Durchschnitts- bzw. einer Hintergrundmenge an p97, die für die Kontrollproben bestimmt wurde, verglichen wird.

4. Verfahren nach Anspruch 1, wobei die Kontrollprobe in b) eine normale Probe vom gleichen Patienten oder eine normale Probe von einer anderen Person ist.

5. Verfahren nach irgendeinem der Ansprüche 1 bis 4, wobei die Menge an p97 in Schritt a) mit Hilfe von Antikörpern gegen p97 bestimmt wird.

## Revendications

1. Méthode pour diagnostiquer la maladie d'Alzheimer par quantification de p97 dans un échantillon test de sérum d'un patient suspecté d'être atteint de la maladie d'Alzheimer comprenant les étapes consistant à :
a) déterminer la quantité de p97 dans l'échantillon test ; et
b) comparer la quantité de p97 dans l'échantillon test avec une quantité de p97 dans les échantillons témoins ;
dans laquelle la présence d'une quantité de p97 dans l'échantillon test qui est une fois et demie plus élevée ou plus comparé à la quantité de p97 dans les échantillons témoins indique le risque de maladie d'Alzheimer.

2. Méthode selon la revendication 1, dans laquelle la quantité de p97 dans l'échantillon test qui est de deux à neuf fois plus élevée comparé à la quantité de p97 dans les échantillons témoins indique le risque de maladie d'Alzheimer.

3. Méthode selon la revendication 1, dans laquelle à l'étape (b), la quantité de p97 dans l'échantillon test est comparée à une quantité moyenne ou de base de p97 déterminée dans les échantillons témoins.

4. Méthode selon la revendication 1, dans laquelle l'échantillon témoin à l'étape (b) est un échantillon normal du même patient ou d'un autre individu.

5. Méthode selon l'une quelconque des revendications 1 à 4, dans laquelle la quantité de p97 à l'étape (a) est déterminée en utilisant des anticorps anti-p97.
